# EUROPÄISCHE PATENTSCHRIFT

(11) **EP 0 763 699 B1**
(45) Veröffentlichungstag und Bekanntmachung des Hinweises auf die Patenterteilung: **01.03.2000**
(21) Anmeldenummer: 96116225.2
(22) Anmeldetag: 21.07.1995
(51) Int. Cl.: F24F 3/12, F24F 1/02, F24F 6/12

(54) **Heilklimagerät**
Curative air conditioner
Appareil de climatisation thérapeutique

(30) Priorität: 23.07.1994 DE 4426218
(43) Veröffentlichungstag der Anmeldung: 19.03.1997
(62) Teilanmeldung aus: 95111496.6
(73) Patentinhaber: Metrax GmbH, D-78628 Rottweil (DE)
(72) Erfinder: Bucher, Heinz, 78628 Rottweil (DE)
(74) Vertreter: Fleck, Hermann-Josef, Dr.-Ing.

(56) Entgegenhaltungen:
- EP-A- 0 522 439
- DE-A- 3 518 456
- DE-A- 3 522 946

## Beschreibung

Die Erfindung betrifft ein Heilklimagerät mit einem ein Gehäuseoberteil und ein Gehäuseunterteil aufweisendem Gehäuse, das einen ein Gebläse aufnehmenden Luftführungskanal aufweist, der über eine Lufteintritts- und eine Luftaustrittsöffnung mit der Umgebung in Verbindung steht.

Ein derartiges Heilklimagerät ist aus der DE 35 18 456 C2 bekannt. Bei diesem bekannten Heilklimagerät wird von einem Gebläse Umgebungsluft durch die Lufteintrittsöffnung in den Luftführungskanal eingesaugt. Die Luft wird noch bevor sie das Gebläse erreicht, an verschiedenen Leuchten vorbeigeführt. Die Leuchten senden verschiedene Spektren von UV-Licht aus, mit dem Mikroorganismen in der Luft abgetötet werden. Hinter dem Gebläse streicht die Luft an einer elektronischen Heizeinrichtung vorbei. Dann wird sie durch einen Wassernebel geleitet. Das Wasser für den Wassernebel ist in einer, am Gehäuseunterteil festgemachten Schale bevorratet. Die durch den Wassernebel hindurchgeführte Luft wird mit Feuchtigkeit und Salzen bzw. ätherischen Ölen angereichert und wieder in die Umgebung durch die Luftaustrittsöffnung abgegeben. Die Steuerung des Heilklimagerätes erfolgt über ein zentrales Leistungsteil.

Da in dem Heilklimagerät Wasser und Elektrizität geleitet wird, ist hohen Sicherheitsanforderungen zu entsprechen. Aus diesem Grund werden die einzelnen elektrischen Einheiten gekapselt. Dazu werden jeweils einzelne Gehäuse verwendet, in die die elektronischen Bauelemente feuchtigkeitsdicht eingebettet sind.

Für die Trennung der stromführenden Teile von dem im Heilklimagerät geleiteten Wasser ist ein hoher Teile- und Montageaufwand notwendig. Auch sind hohe Anforderungen an den Monteur eines solchen Heilklimagerätes zu stellen, denn er muß sorgfältig die einzelnen elektronischen Bauelemente wassergeschützt einbauen. Anschließend muß er, ebenfalls wassergeschützt, die Verdrahtung vornehmen.

Es ist die Aufgabe der Erfindung, ein Heilklimagerät der eingangs erwähnten Art zu schaffen, das bei geringem Teileaufwand eine einfache Montage ermöglicht.

Diese Aufgabe wird dadurch gelöst, daß in dem Gehäuse ein Zwischengehäuseteil gebildet ist, das ein Zwischengehäuseoberteil und ein Zwischengehäuseunterteil aufweist und einen Aufnahmeraum für elektronische Bauelemente feuchtigkeitsdicht umschließt.

Das Zwischengehäuseteil kann als separate Baugruppe außerhalb des Heilklimagerätes vormontiert werden. In dem Zwischengehäuseteil werden die elektronischen Bauelemente eingebaut. Hierzu läßt sich das Zwischengehäuseteil in ein Zwischengehäuseoberteil und in ein -unterteil trennen. Der Aufnahmeraum für die elektronischen Bauelemente ist dann leicht zugänglich. Wenn die Bauelemente eingebaut und verdrahtet sind, dann kann das Zwischengehäuseteil zusammengebaut und abgedichtet werden.

Nach einer Ausgestaltung der Erfindung ist vorgesehen, daß der Luftführungskanal zwischen dem Gehäuseoberteil und dem Gehäuseunterteil gebildet ist. Damit kann auf separate Bauteile für die Luftführung verzichtet werden.

Zur Anbringung des Gebläses kann vorgesehen sein, daß das Gebläse ein auf einem Halteabschnitt des Zwischengehäuseoberteils angeordnetes Verdichterrad aufweist, und daß das Verdichterrad in dem Luftführungskanal unter einem Luftfilterkasten angeordnet ist.

Ist vorgesehen, daß die Lufteintrittsöffnung des Luftführungskanals nach oben abgedeckt ist, so daß die Luft entgegen der Schwerkraftrichtung in den Luftführungskanal eingesaugt wird, dann ist auf einfache Weise verhindert, daß bei Nichtbetrieb des Heilklimagerätes Verschmutzungen in dem Luftführungskanal in Folge von in der Umgebung absinkenden Staubteilen auftreten.

Nach einer bevorzugten Ausgestaltung der Erfindung ist vorgesehen, daß der Luftführungskanal mittels einer Trennwand in zwei Bereiche unterteilt ist, daß die Trennwand eine Ausnehmung aufweist, die die beiden Bereiche miteinander verbindet, daß der in Luftströmungsrichtung vor der Trennwand liegende erste Bereich das Verdichterrad und der in Luftströmungsrichtung hinter der Trennwand liegende zweite Bereich die UV-Lampe und eine als Brauserohr ausgebildete Sprühvorrichtung aufnimmt und daß der zweite Bereich Luftaustrittsöffnungen aufweist und mit einem einen Wasservorrat aufnehmenden Gehäuseunterteil in räumlicher Verbindung steht.

Um das Zwischengehäuse abzustützen, kann vorgesehen sein, daß das Zwischengehäuseunterteil nach unten abstehende Standfüße aufweist, und daß das Zwischengehäuseteil in das Gehäuseunterteil eingestellt ist.

Eine mögliche Erfindungsvariante ist dadurch gekennzeichnet, daß die elektrischen Bauelemente auf einer Grundplatte montiert sind, und daß die Grundplatte mit dem Halteabschnitt des Zwischengehäuseoberteils verbunden ist. Auf der Grundplatte können die elektronischen Bauelemente montiert werden. Die Grundplatte läßt sich dann in das Zwischengehäuse einbauen.

Die Erfindung wird im folgenden anhand eines in den Zeichnungen dargestellten Ausführungsbeispiels näher erläutert. Es zeigen:
- Fig. 1: in perspektivischer Ansicht ein Heilklimagerät mit einem Gehäuseoberteil und einem Gehäuseunterteil,
- Fig. 2: das Heilklimagerät nach Fig. 1 mit von dem Gehäuseoberteil abgenommenem Deckel und
- Fig. 3: das Heilklimagerät nach den Fig. 1 und 2 in Seitendarstellung und im Schnitt.

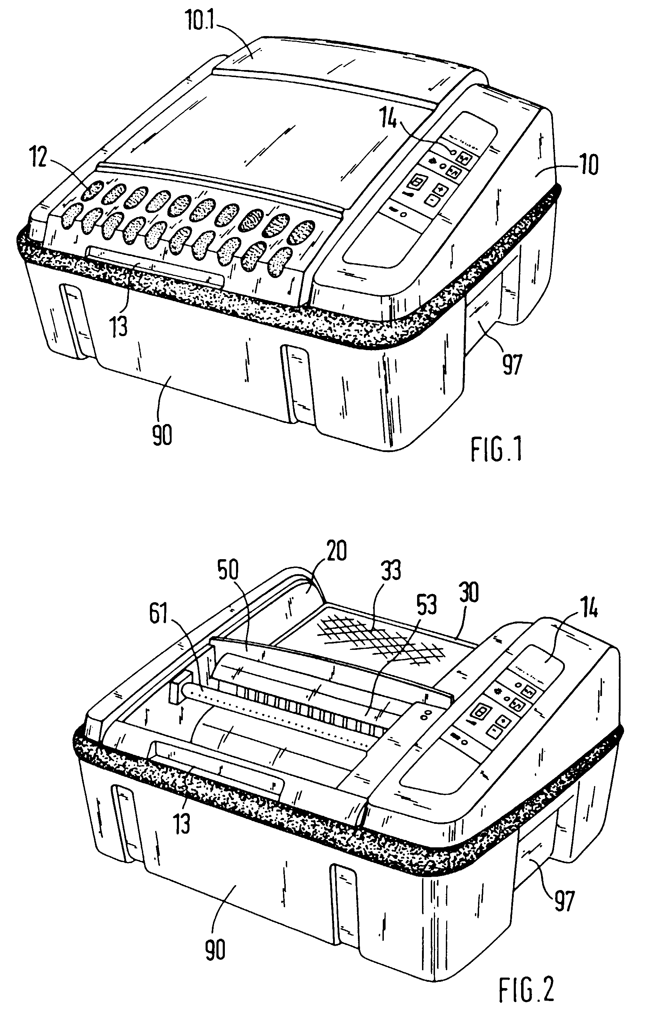

Die Fig. 1 zeigt ein Heilklimagerät mit einem Gehäuseunterteil 90 und einem Gehäuseoberteil 10. Das Gehäuseoberteil 10 weist einen Deckel 10.1 auf, der mit Luftaustrittsöffnungen 12 versehen ist. Zum Bedienen des Heilklimagerätes ist neben dem Deckel 10.1 eine Bedieneinheit 14 angeordnet. Zum Transport des Heilklimagerätes sind seitlich in das Gehäuseunterteil 90 Griffmulden 97 eingeformt. Der Deckel 10.1 läßt sich nach Aufheben einer Verriegelung 13 von dem Gehäuseoberteil 10 abheben.

Die Fig. 2 zeigt das Heilklimagerät mit abgehobenem Deckel 10.1. Wie aus dieser Darstellung ersichtlich ist, deckt der Deckel ein Filterelement 33, eine Trennwand 50 mit angeformter Abdeckung 53 sowie ein Brauserohr 61 ab. Unter der Abdeckung 53 ist eine in dieser Darstellung nicht ersichtliche UV-Lampe 60 angeordnet.

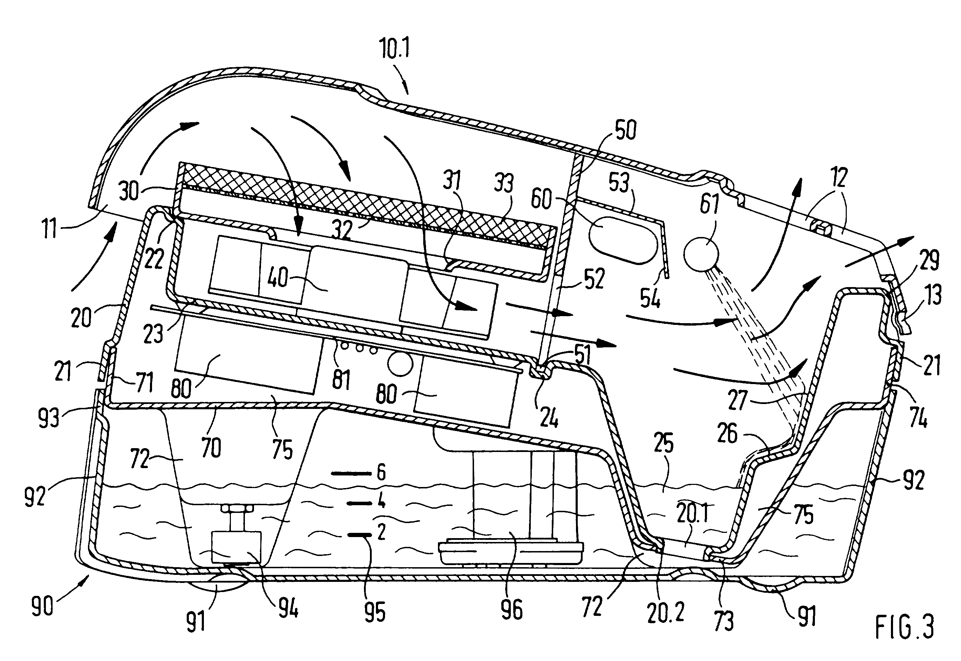

Die Fig. 3 zeigt in Seitendarstellung und im Schnitt das Heilklimagerät. Zwischen dem Gehäuseoberteil 10 und dem Gehäuseunterteil 90 ist ein Zwischengehäuse angeordnet, das im wesentlichen aus einem Zwischengehäuseoberteil 20 und einem Zwischengehäuseunterteil 70 gebildet ist. Das Zwischengehäuseoberteil 20 ist mit einem umlaufenden abgekröpften Rand 21 versehen. Mit diesem abgekröpften Rand 21 ist das Zwischengehäuseoberteil 20 auf einen nach oben abgewinkelten Rand 71 des Zwischengehäuseunterteils 70 aufgesetzt. Die Verbindung des Zwischengehäuseoberteils 20 mit dem Zwischengehäuseunterteil 70 erfolgt mittels Schraubverbindungen. Hierzu sind Befestigungsschrauben in den abgekröpften Rand 21 und den abgewinkelten Rand 71 eingeschraubt. Zwischen dem abgekröpften Rand 21 und dem abgewinkelten Rand 71 kann ein Dichtring eingelegt sein. Zwischen dem Zwischengehäuseoberteil 20 und dem Zwischengehäuseunterteil 70 ist ein Zwischenraum 75 gebildet, in dem elektrische Bauelemente 80 untergebracht sind.

Die elektrischen Bauelemente 80 sind auf einer Grundplatte 80 montiert. Die Grundplatte 81 wiederum ist mit einem Halteabschnitt 23 des Zwischengehäuseoberteils 20 verbunden. An das Zwischengehäuseunterteil 70 sind nach unten abstehende Standfüße 72 einstückig angeformt. Das aus dem Zwischengehäuseoberteil 20 und dem Zwischengehäuseunterteil 70 gebildete Zwischengehäuse ist in das Gehäuseunterteil 90 eingestellt. Hierbei stützen sich die Standfüße 72 auf dem Boden des Gehäuseunterteils 90 ab. Das Zwischengehäuse stützt sich zur Vorderseite mit seinem Zwischengehäuseunterteil 70 an einer Seitenwand 92 des Gehäuseunterteils 90 ab. Das Zwischengehäuse stützt sich zur Vorderseite mit seinem Zwischengehäuseunterteil 70 an einer Seitenwand 92 des Gehäuseunterteils 90 ab. Rückseitig weist die Seitenwand 92 einen abgekröpften Rand 93 auf, an dem sich das Zwischengehäuse 70 anstützt. Damit ist das Zwischengehäuse zentriert in dem Gehäuseunterteil 90 gehalten.

Das Gehäuseunterteil 90 dient zur Aufnahme eines Wasservorrates. In den den Wasservorrat aufnehmenden Bereich des Gehäuseunterteils 90 ragt eine Pumpe 96 mit ihrem Ansaugrohr. Die Pumpe 96 ist an dem Zwischengehäuseunterteil 70 festgelegt. Zur Detektierung des Wasserstandes ist ein Niveaugeber 94 verwendet, der an einem der Standfüße 72 montiert ist. Zwischen dem Deckel 10.1 des Gehäuseoberteils 10 und dem Zwischengehäuseoberteil 20 ist ein Luftführungskanal gebildet. Der Luftführungskanal steht über eine Lufteintrittsöffnung 11 und Luftaustrittsöffnung 12 mit der Umgebung in Verbindung. Die Lufteintrittsöffnung 11 ist zwischen dem Deckel 10.1 und dem Zwischengehäuseoberteil 20 gebildet. Hierzu steht der Deckel 10.1 rückseitig über das Zwischengehäuseoberteil 20 vor.

Die Lufteintrittsöffnung 11 ist nach unten geöffnet, so daß bei Nichtbetrieb des Heilklimagerätes kein Staub in den Luftführungskanal eindringen kann. Der Luftführungskanal ist mittels der Trennwand 60 in zwei Bereiche unterteilt. In dem ersten Bereich, der der Lufteintrittsöffnung 11 zugeordnet ist, ist ein ein Verdichterrad 40 aufweisendes Gebläse eingebracht. Das Gebläse ist auf dem Halteabschitt 23 befestigt. Oberhalb des Verdichterrades 40 weist das Zwischengehäuseoberteil 20 eine Luftfilterkastenaufnahme 22 auf. Auf diese Luftfilderkastenaufnahme 22 ist ein Luftfilterkasten 30 aufgesetzt. In dem Luftfilterkasten 30 ist ein Rost 32 befestigt. Der Rost 32 trägt ein Filterelement 33. In dem zweiten, den Luftaustrittsöffnungen 12 zugeordneten Bereich ist die UV-Lampe 60 und eine als Brauserohr 61 ausgebildete Sprühvorrichtung angeordnet. Die Trennwand 50 trägt eine Abdeckung 53, die die UV-Lampe 60 überdeckt. Hierbei verhindert der Schenkel 54 der Abdeckung 53, daß Licht, das von der UV-Lampe 60 ausgesandt wird, durch die Luftaustrittsöffnungen 12 des Deckels 10.1 in die Umgebung gelangt.

Unterhalb der Abdeckung 53 und der UV-Lampe 60 weist die Trennwand 50 einen Durchbruch 52 auf, mittels dem der erste und der zweite Bereich des Luftführungskanals in Verbindung stehen. Die Trennwand 50 ist mit einem Steckansatz 51 in einem Schlitz 24 des Zwischengehäuseoberteils 20 eingesetzt.

Das Gebläse saugt mit seinem Verdichterrad 40 Umgebungsluft durch die Lufteintrittsöffnung 11 an. Die angesaugte Luft tritt durch das Filterelement 33 und den Rost 32 in Achsrichtung der Drehachse in das Verdichterrad 40 ein. Im Verdichterrad 40 wird der Luftstrom um 90° umgelenkt und radial nach außen über den Umfang des Verdichterrades 40 wieder abgegeben. Die Trennwand 50, der Luftfilterkasten 30, der Halteabschnitt 23 und der an den Halteabschnitt 23 anschließende, nach oben gerichtete Steg des Zwischengehäuseoberteils 20 bilden ein Gehäuse, innerhalb dessen das Verdichterrad 40 angeordnet ist.

Damit wird die von dem Verdichterrad 40 ausgeblasene Luft durch den Durchbruch 52 der Trennwand 50 in den zweiten Bereich des Luftführungskanals geleitet. Nach dem Durchtritt durch den Durchbruch 52 wird die Luft mit der UV-Lampe 60 bestrahlt. Hierbei werden Keime, die mit der Luft mitgeführt werden, abgetötet. Anschließend wird die Luft durch einen von dem Brauserohr 61 erzeugten Wasservorhang hindurchgeleitet. Hierbei nimmt die Luft Feuchtigkeit auf und strömt anschließend durch die Luftaustrittsöffnung 12 wieder in die Umgebung ab.

Dem Brauserohr 61 wird das Wasser über ein Kanalsystem von der Pumpe 96 zugeführt. Das nicht von der Luft aufgenommene übrige Wasser wird von einer Prallwand 27 des Zwischengehäuseoberteils 20 abgefangen und über eine Schräge 26 nach unten abgeleitet. Von hier gelangt es in eine Vertiefung 25, die von dem Zwischengehäuseoberteil 20 gebildet ist. Die Vertiefung 25 steht über einen Durchbruch 20.1 mit dem im Gehäuseuntereil 90 aufgenommenen Wasservorrat in Verbindung. Das nicht von dem Luftstrom mitgeführte Wasser kann somit wieder in den Wasservorrat des Gehäuseunterteils 90 und steht erneut der Pumpe 96 zur Verfügung. An dem Durchbruch 20.1 ist ein ringförmiger Ansatz 20.2 angeschlossen, der in eine Ausnehmung 73 des Zwischengehäuseunterteils 70 eingesteckt ist. Der ringförmige Ansatz 20.2 ist gegen das Zwischengehäuseunterteil 70 mittels einer Ringdichtung abgedichtet. Zur Verringerung der Schallemission leitet das Brauserohr 61 das abgesprühte Wasser im spitzen Winkel gegen die Prallwand 27.

Die Reinigung des Gerätes ist einfach möglich. Hierzu muß der Deckel 10.1 abgenommen werden. Dies ist dadurch möglich, daß die an der Vorderseite gebildete Verriegelung 13 aufgehoben wird. Hierzu ist ein Griff vorgesehen, mittels dem der Deckel nach oben gezogen werden kann, wobei die Verriegelung 13 elastisch an der Kante 29 des Zwischengehäuseoberteils 20 zurückfedert. Bei abgenommenem Deckel 10.1 ist das Filterelement 33 zugänglich. Zum Auswechseln kann es einfach aus dem Luftfilterkasten 30 vom Rost 32 abgenommen und gegen ein neues Filterelement 33 ausgetauscht werden. Zur Reinigung des zweiten Bereiches des Luftführungskanals kann die Trennwand 50 nach oben aus dem Gehäuse herausgezogen werden. Hierdurch wird die UV-Lampe 60 freigelegt und kann mit einem Tuch gereinigt oder im Schadensfall einfach ausgetauscht werden. Das Brauserohr 61 ist herausnehmbar von oben in Aufnahmen eingesetzt. Es läßt sich einfach herausziehen und von außen und innen mit einer Bürste reinigen. Um sicherzustellen, daß nach dem erneuten Einbau des Brauserohres 61 der Sprühwinkel gegen die Prallwand 27 erhalten bleibt, sind an den das Brauserohr 61 haltenden Aufnahmen Führungen vorgesehen, die nur eine einzige Einbaulage zulassen.

Zum Nachfüllen des Wasservorrates bzw. zum Reinigen des Gehäuseunterteils 90 kann die aus dem Gehäuseoberteil 10, Zwischengehäuseoberteil 20 und Zwischengehäuseunterteil 70 bestehende Einheit von dem Gehäuseunterteil 90 abgehoben werden. Dann kann das Gehäuseunterteil 90 gereinigt oder Wasser aufgefüllt werden. Eine Wasserstandsanzeige 95 am Gehäuseunterteil 90 zeigt den aktuellen Füllstand.

Die UV-Lampe 60 strahlt in die Vertiefung 25 des Zwischengehäuseoberteils 25 ein. Damit bestrahlt sie auch den in dem Gehäuseunterteil 90 aufgenommenen Wasservorrat. Hierdurch wird eine Entkeimung des Wassers erzielt. Vorteilhafterweise beginnt nach dem Einschalten des Gerätes auch ein zeitlich begrenzter Entkeimungsvorgang, wobei die UV-Lampe 60 den Wasservorrat entkeimt. In diesem Betriebszustand ist das Gebläse noch nicht in Betrieb. Nach Ablauf des Entkeimungsvorganges startet das Gerät. Zur Anregung der vom Wasserdampf mitgeführten Flüssigkeitströpfchen wird ein in den Zeichnungen nicht dargestellter Schumann-Wellengenerator verwendet. Dieser erzeugt eine Schwingungsfrequenz, die sich auf die Wassertropfen überträgt. Die Frequenz einer Schumann-Welle entspricht im übrigen einer Schwingung von 7,8 Hz, die auch verstärkt in Meeresnähe meßbar ist.

Da die Schumann-Welle sehr niederfrequent sind, muß darauf geachtet werden, daß die durch die Elektronik des Heilklimagerätes erzeugten Störimpulse die Schumann-Wellen nicht neutralisieren. Dies wird vorliegend dadurch erreicht, daß sowohl die Pumpe 96, als auch das Gebläse mit einer Null-Durchgangssteuerung betrieben werden. Für das Gebläse ist ein kommuntierter Gleichstrommotor verwendet. Hierdurch wird eine nur geringe Magnetfelderzeugung bewirkt.

## Patentansprüche

1. Heilklimagerät mit einem ein Gehäuseoberteil (10) und ein Gehäuseunterteil (90) aufweisenden Gehäuse, das einen ein Gebläse (40) aufnehmenden Luftführungskanal aufweist, der über eine Lufteintritts- und eine Luftaustrittsöffnung (11;12) mit der Umgebung in Verbindung steht,
dadurch gekennzeichnet,
daß in dem Gehäuse ein Zwischengehäuseteil gebildet ist, das ein Zwischengehäuseoberteil (20) und ein Zwischengehäuseunterteil (70) aufweist und einen Aufnahmeraum (75) für elektronische Bauelemente (80) feuchtigkeitsdicht umschließt.

2. Heilklimagerät nach Anspruch 1,
dadurch gekennzeichnet,
daß der Luftführungskanal zwischen dem Gehäuseoberteil (10) und dem Gehäuseunterteil (20) gebildet ist.

3. Heilklimagerät nach Anspruch 1 oder 2,
dadurch gekennzeichnet,
daß das Gebläse ein auf einem Halteabschnitt (23) des Zwischengehäuseoberteils (20) angeordnetes Verdichterrad (40) aufweist, und
daß das Verdichterrad (40) in dem Luftführungskanal unter einem Luftfilterkasten (31) angeordnet ist.

4. Heilklimagerät nach einem der Ansprüche 1 bis 3,
dadurch gekennzeichnet,
daß die Luftaustrittsöffnung (11) des Luftführungskanals nach unten gerichtet ist, so daß die Luft entgegen der Schwerkraftrichtung in den Luftführungskanal eingesaugt wird.

5. Heilklimagerät nach einem der Ansprüche 1 bis 4,
dadurch gekennzeichnet,
daß der Luftführungskanal mittels einer quer zur Luftströmungsrichtung angeordneten Trennwand (50) in zwei Bereiche unterteilt ist, daß die Trennwand (50) eine Ausnehmung (52) aufweist, die die beiden Bereiche miteinander verbindet,
daß der in Luftströmungsrichtung vor der Trennwand (50) liegende erste Bereich das Verdichterrad (40) und der in Luftströmungsrichtung hinter der Trennwand liegende zweite Bereich eine UV-Lampe (60) und eine als Brauserohr (61) ausgebildete Sprühvorrichtung aufnimmt, und
daß der zweite Bereich die Luftaustrittsöffnungen 812) aufweist und mit einem, einen Wasservorrat aufnehmenden Gehäuseunterteil (90) in räumlicher Verbindung steht.

6. Heilklimagerät nach einem der Ansprüche 1 bis 5,
dadurch gekennzeichnet,
daß das Zwischengehäuseunterteil (70) nach unten abstehende Standfüße (72) aufweist, und
daß das Zwischengehäuseteil in das Gehäuseunterteil (90) eingestellt ist.

7. Heilklimagerät nach einem der Ansprüche 1 bis 6,
dadurch gekennzeichnet,
daß die elektrischen Bauelemente (80) auf einer Grundplatte (81) montiert sind, und
daß die Grundplatte (81) mit dem Halteabschnitt (23) des Zwischengehäuseoberteils (20) verbunden ist.

8. Heilklimagerät nach einem der Ansprüche 1 bis 7,
dadurch gekennzeichnet,
daß das Zwischengehäuseoberteil (20) in dem zweiten Bereich eine Vertiefung (25) aufweist, die in dem Zwischengehäuseoberteil (20) gebildet ist, und
daß die Vertiefung (25) mit dem Gehäuseunterteil (90) über einen Durchbruch (20.1) und einen daran angeschlossenen Ansatz (20.2) in Verbindung steht, der in eine Ausnehmung (73) des Zwischengehäuseunterteils (70) eingesteckt ist.

## Claims

1. Therapeutic air conditioner, having a housing, which includes an upper housing portion (10) and a lower housing portion (90) and includes an air guiding duct, which accommodates a fan (40) and which communicates with the ambient surroundings via an air inlet aperture (11) and an air outlet aperture (12), characterised in that an intermediate housing portion is formed in the housing, which portion includes an upper intermediate housing portion (20) and a lower intermediate housing member (70) and surrounds a receiving chamber (75) for electronic components (80) in a moisture-tight manner.

2. Therapeutic air conditioner according to claim 1, characterised in that the air guiding duct is formed between the upper housing portion (10) and the lower housing portion (20).

3. Therapeutic air conditioner according to claim 1 or 2, characterised in that the fan includes a compressor impeller (40), which is disposed on a retaining portion (23) of the upper intermediate housing portion (20), and in that the compressor impeller (40) is disposed in the air guiding duct beneath an air filter box (30).

4. Therapeutic air conditioner according to one of claims 1 to 3, characterised in that the air inlet aperture (11) of the air guiding duct is orientated downwardly, so that the air is drawn by suction into the air guiding duct in a direction opposite the direction of gravitational force.

5. Therapeutic air conditioner according to one of claims 1 to 4, characterised in that the air guiding duct is divided into two regions by means of a dividing wall (50) disposed transversely relative to the air flow direction, in that the dividing wall (50) includes a recess (52), which interconnects the two regions, in that the first region, situated in front of the dividing wall (50), when viewed with respect to the air flow direction, accommodates the compressor impeller (40), and the second region, situated behind the dividing wall, when viewed with respect to the air flow direction, accommodates a UV lamp (60) and a spraying device, which is configured as a sprinkler tube (61), and in that the second region includes the air outlet apertures (12) and communicates physically with a lower housing portion (90), which accommodates a water supply.

6. Therapeutic air conditioner according to one of claims 1 to 5, characterised in that the lower intermediate housing portion (70) includes downwardly protruding supporting feet (72), and in that the intermediate housing portion is fitted into the lower housing portion (90).

7. Therapeutic air conditioner according to one of claims 1 to 6, characterised in that the electrical components (80) are mounted on a base plate (81), and in that the base plate (81) is connected to the retaining portion (23) of the upper intermediate housing portion (20).

8. Therapeutic air conditioner according to one of claims 1 to 7, characterised in that the upper intermediate housing portion (20) has, in the second region, an indentation (25) which is formed in the upper intermediate housing portion (20), and in that the indentation (25) communicates with the lower housing portion (90) via an opening (20.1) and an exension member (20.2), which communicates with said opening and is inserted into a recess (73) in the lower intermediate housing portion (70).

## Revendications

1. Appareil de climatisation thérapeutique avec un boîtier comprenant un élément de boîtier supérieur (10) et un élément de boîtier inférieur (90), qui présente un canal de guidage d'air contenant une soufflante (40), canal qui par l'intermédiaire d'une ouverture d'admission d'air et d'une ouverture d'échappement d'air (11; 12) est en communication avec l'atmosphère ambiante,
caractérisé en ce que dans le boîtier est formé un élément de boîtier intermédiaire, qui présente un élément de boîtier intermédiaire supérieur (20) et un élément de boîtier intermédiaire inférieur (70) et qui entoure un espace de réception (75) pour des composants électroniques (80).

2. Appareil de climatisation thérapeutique suivant la revendication 1,
caractérisé
en ce que le canal de guidage d'air est formé entre l'élément de boîtier supérieur (10) et l'élément de boîtier inférieur (20).

3. Appareil de climatisation thérapeutique suivant la revendication 1 ou la revendication 2,
caractérisé
en ce que la soufflante présente une roue de compresseur (40) disposée sur un tronçon de fixation (23) de l'élément de boîtier supérieur (20), et
en ce que la roue de compresseur (40) dans le canal de guidage d'air est disposée au-dessous d'un caisson de filtre d'air (31).

4. Appareil de climatisation thérapeutique suivant l'une quelconque des revendications de 1 à 3,
caractérisé
en ce que l'ouverture d'échappement d'air (11) du canal de guidage d'air est dirigé vers le dessous, de sorte que l'air est aspiré dans le canal de guidage d'air à l'encontre du sens de la force de gravité.

5. Appareil de climatisation thérapeutique suivant l'une quelconque des revendications de 1 à 4,
caractérisé
en ce que le canal de guidage d'air est, au moyen d'une cloison de séparation (50) disposée transversalement à la direction de circulation d'air, partagé en deux zones,
en ce que la cloison de séparation (50) présente un évidement (52), qui met en communication les deux zones,
en ce que la première zone qui dans le sens de la circulation d'air est située devant la cloison de séparation reçoit la roue de compresseur (40) tandis que la deuxième zone, à savoir celle qui est située derrière la cloison de séparation, reçoit une lampe UV (60) et un dispositif de pulvérisation ayant la forme d'une pomme de douche (61), et en ce que la deuxième zone présente les ouvertures d'échappement d'air (12) et est en communication spatiale avec un élément de boîtier inférieur (90) contenant une réserve d'eau.

6. Appareil de climatisation thérapeutique suivant l'une quelconque des revendications de 1 à 5,
caractérisé
en ce que l'élément de boîtier intermédiaire inférieur (70) présente des pieds de support faisant protubérance vers le dessous, et
en ce que l'élément de boîtier intermédiaire inférieur est placé dans l'élément de boîtier inférieur (90).

7. Appareil de climatisation thérapeutique suivant l'une quelconque des revendications de 1 à 6,
caractérisé
en ce que les composants électroniques (80) sont montés sur une platine de base (81), et
en ce que la platine de base (81) est reliée au tronçon de fixation (23) de l'élément de boîtier intermédiaire supérieur (20).

8. Appareil de climatisation thérapeutique suivant l'une quelconque des revendications de 1 à 7,
caractérisé
en ce que l'élément de boîtier intermédiaire supérieur (20) dans la seconde zone présente un renfoncement (25), qui est formé dans l'élément de boîtier intermédiaire supérieur (20),et
en ce que le renfoncement (25) est par l'intermédiaire d'un percement (20.1) et d'un épaulement (20.2) y raccordé en communication avec l'élément de boîtier inférieur (90), l'épaulement étant enfiché dans un évidement (73) de l'élément de boîtier intermédiaitre inférieur (70).
